# FASCICULE DE BREVET EUROPEEN

(11) **EP 2 676 637 B1**
(45) Date de publication et mention de la délivrance du brevet: **18.09.2019**
(21) Numéro de dépôt: 13172776.0
(22) Date de dépôt: 19.06.2013
(51) Int. Cl.: A61F 2/38, A61F 2/30

(54) **Ensemble d'implants fémoraux pour une prothèse du genou**
Satz von Femoralimplantaten für eine Knieprothese
Femoral implant set for a knee prosthesis

(30) Priorité: 20.06.2012 FR 1255798
(43) Date de publication de la demande: 25.12.2013
(73) Titulaire: Corin Limited, Cirencester Gloucestershire GL7 1YJ (GB)
(72) Inventeur: Faure, Eric, 38190 Laval (FR); Goubet, Nicolas, 38190 Bernin (FR); Dejour, David, 69006 Lyon (FR); Erasmus, Spike, Stellenbosch - Western Cape (ZA); ARENDT, Elizabeth, Minneapolis, MN 55454 (US); Feller, Julian, Camberwell, Victoria (AU); Almqvist, Fredrik, 9000 Gent (BE)
(74) Mandataire: Schuffenecker, Thierry

(56) Documents cités:
- FR-A1- 2 728 782
- FR-A1- 2 908 040
- FR-A1- 2 955 482
- US-A- 5 226 915
- US-A1- 2012 041 566

## Description

La présente invention concerne un ensemble d'implants fémoraux pour une prothèse du genou. Le domaine de l'invention est celui des prothèses de genou, mises en oeuvre lorsque le fémur d'un patient nécessite une prothèse fémoro-patellaire.

L'invention s'applique en particulier aux prothèses unicompartimentales De manière connue, une telle prothèse comprend un implant fémoral adapté pour coopérer avec un implant rotulien ou une rotule native. L'implant fémoral comprend une face externe dont le profil permet une cinématique du genou prothésé proche de la cinématique originelle du genou, ainsi qu'une face interne dont le profil est critique pour le positionnement et la fixation de l'implant au fémur. Une fois posé, cet implant est soumis à des contraintes mécaniques importantes, sollicitant notamment la fixation sur l'os, durant les différentes phases de fonctionnement : marche, accroupissement, etc.

Ainsi, il est essentiel que l'implant fémoral soit positionné avec précision sur l'os qui nécessite une réparation, puis immobilisé de manière rigide et durable. Ces fonctions sont notamment assurées par la mise en oeuvre de moyens d'ancrage osseux formés sur la face interne de l'implant, et par l'interposition de ciment chirurgical entre la face interne et les surfaces de préparation osseuse du fémur.

Avant d'implanter la prothèse au patient, le chirurgien effectue une préparation osseuse qui correspond, d'une part, à l'anatomie du patient et, d'autre part, à un profil d'implant disponible. Plus précisément, l'implantation d'une telle prothèse nécessite la réalisation de préparations osseuses distales et antérieure du fémur. Dans ces conditions, il est avantageux de disposer d'un ensemble d'implants fémoraux présentant des profils et dimensions différentes, afin de choisir l'implant le mieux adapté à l'anatomie du patient.

Toutefois, des implants différents peuvent nécessiter une modification de la préparation osseuse avant que l'implant adapté puisse être implanté, ce qui génère une perte de temps et peut nuire à la qualité de l'intervention chirurgicale. En particulier, l'implantation de la prothèse est insatisfaisante et peut avoir des conséquences néfastes si, du fait de son profil et ses dimensions, l'implant n'est pas positionné dans une configuration affleurant les surfaces de préparation osseuse.

FR-A-2 908 040 décrit un ensemble d'implants fémoraux pour une prothèse unicompartimentale du genou. Ces implants sont symétriques et destinés à remplacer uniquement l'un des deux compartiments, interne ou externe, du fémur. Ainsi, ces implants présentent une forme relativement simple en comparaison avec un implant bicompartimental. Les épaisseurs de ces implants, considérées au droit des surfaces d'appui internes distale et postérieure, sont égales et constantes quelles que soient les dimensions de l'implant, tandis que les longueurs des surfaces d'appui distale et postérieure peuvent varier entre les implants. Autrement dit, les parties distales et postérieures des implants présentent des dimensions différentes pour chaque implant, ce qui n'est pas satisfaisant.

US-A-5 226 915, FR-A-2 728 782 et FR-A-2 955 482 décrivent d'autres exemples d'implants fémoraux.

Le but de la présente invention est de proposer un ensemble d'implants fémoraux améliorés.

A cet effet, l'invention a pour objet un ensemble d'implants fémoraux pour une prothèse de genou selon la revendication 1.

Ainsi, l'invention permet de simplifier la préparation osseuse, de faciliter le positionnement de l'implant et d'améliorer sa tenue en service. Grâce à l'invention, au moins la préparation osseuse distale est identique pour tous les implants appartenant à l'ensemble, quel que soit le choix d'implant du chirurgien. La préparation distale initiale correspond au plus petit implant de l'ensemble. La bordure distale de l'implant affleure la bordure de la préparation osseuse distale, autrement dit l'implant est positionné à quai sur l'os. Ceci permet d'obtenir une bonne transition os-implant et évite tout ressaut de la rotule à la jonction de l'implant et de l'os lors d'un mouvement de flexion vers l'extension ou d'extension vers la flexion. La course de la rotule est améliorée, ce qui évite tout bruit lors du passage de la rotule entre la prothèse et les condyles distaux cartilagineux et empêche l'usure de l'implant rotulien ou de la rotule native.

De préférence, en plus de la partie distale, d'autres parties des implants constituant l'ensemble présentent les mêmes dimensions et/ou le même agencement d'un implant à l'autre, comme détaillé ci-après.

Selon d'autres caractéristiques avantageuses de l'ensemble d'implants fémoraux selon l'invention, prises isolément ou en combinaison :
- La partie distale de chaque implant fémoral comporte une bordure latérale et une bordure médiale qui, en projection dans un plan anatomique horizontal, s'étendent suivant des droites délimitant un angle distal compris entre 60 degrés et 80 degrés, de préférence compris entre 68 degrés et 72 degrés, en particulier égal à 70 degrés, les angles distaux des implants fémoraux étant identiques, quelles que soient les dimensions des implants fémoraux.
- Les moyens d'ancrage des implants fémoraux présentent des dimensions et un positionnement relatif qui sont identiques, quelles que soient les dimensions des implants fémoraux.
- Chaque implant fémoral comprend une partie intermédiaire reliant la partie distale et la partie antérieure du côté de la face interne, les parties intermédiaires des implants fémoraux présentant des dimensions et un positionnement par rapport à la partie distale qui sont identiques, quelles que soient les dimensions des implants fémoraux.
- Pour chaque implant fémoral, la partie intermédiaire comporte une surface intermédiaire et un bloc chanfrein qui est plus incliné vers la partie distale que la surface intermédiaire.
- Pour chaque implant fémoral, les moyens d'ancrage comprennent au moins un plot distal et au moins un plot antérieur qui sont formés du côté de la face interne, respectivement sur la partie distale et sur la partie antérieure, les plots des implants fémoraux présentant des dimensions et un positionnement relatif qui sont identiques, quelles que soient les dimensions des implants fémoraux.
- Pour chaque implant fémoral, le plot distal et le plot antérieur sont inclinés l'un vers l'autre du côté de la face interne selon un angle aigu compris entre 5 degrés et 15 degrés en projection dans un plan anatomique sagittal.
- Pour chaque implant fémoral, les moyens d'ancrage comportent trois plots antérieurs ayant des axes parallèles entre eux.
- Chaque implant fémoral comporte une surface interne distale et une surface interne frontale qui sont inclinées l'une par rapport à l'autre selon un angle obtus compris entre 90 degrés et 100 degrés, de préférence entre 94 degrés et 96 degrés, en projection dans un plan anatomique sagittal.
- Chaque implant fémoral est unicompartimental et asymétrique.

L'invention concerne également une méthode chirurgicale d'implantation d'une prothèse de genou à un patient, la prothèse comprenant un implant fémoral choisi parmi un ensemble d'implants fémoraux tel que mentionné ci-dessus, cette méthode comprenant :
- une étape de préparation de l'os du fémur, consistant à réaliser au moins une coupe antérieure et une préparation distale ;
- une étape de sélection d'un implant fémoral, dont les dimensions correspondent à l'os du fémur, parmi l'ensemble d'implants fémoraux ; et
- une étape d'implantation de l'implant fémoral sur l'os du fémur, la partie distale affleurant la préparation distale quelles que soient les dimensions de cet implant fémoral.

Selon d'autres caractéristiques avantageuses de la méthode chirurgicale selon l'invention, prises isolément ou en combinaison :
- La méthode comprend également une étape de préparation de la face interne de l'implant fémoral et/ou de l'os du fémur à l'aide d'un ciment osseux chirurgical, avant l'étape d'implantation de l'implant fémoral sur l'os du fémur.
- La méthode comprend également une étape de prise du ciment, après l'étape d'implantation de l'implant fémoral sur l'os du fémur.
- La méthode comprend également une étape de finition et/ou de contrôle et/ou d'équilibrage et/ou de test.

L'invention sera mieux comprise à la lecture de la description qui va suivre, donnée uniquement à titre d'exemple non limitatif et faite en référence aux dessins annexés sur lesquels :
- la figure 1 est une vue en perspective d'un implant fémoral, appartenant à un ensemble d'implants fémoraux conformes à l'invention et destiné à équiper le fémur droit d'un patient ;
- la figure 2 est un autre vue en perspective de l'implant fémoral de la figure 1 ;
- les figures 3 et 4 sont des vues en élévation, respectivement selon les flèches III et IV à la figure 2 ;
- les figures 5 et 6 sont des vues analogues respectivement aux figures 3 et 4, montrant en superposition un ensemble d'implants fémoraux conformes à l'invention ;
- la figure 7 est une vue en élévation selon la flèche VII à la figure 6 ; et
- la figure 8 est une vue en perspective montrant un implant fémoral, appartenant à l'ensemble conforme à l'invention, ancré sur le fémur droit d'un patient.

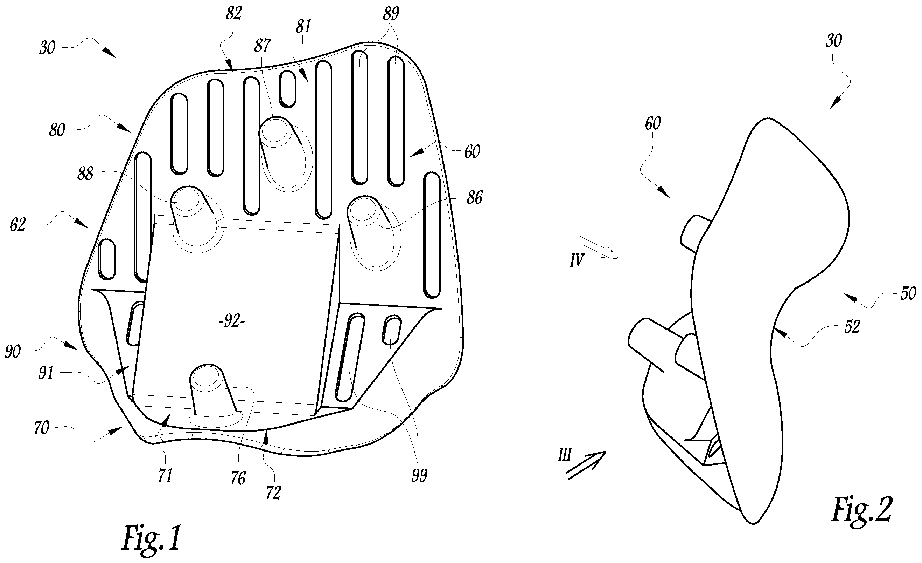

Sur les figures 1 à 8 est représenté un implant fémoral 30.

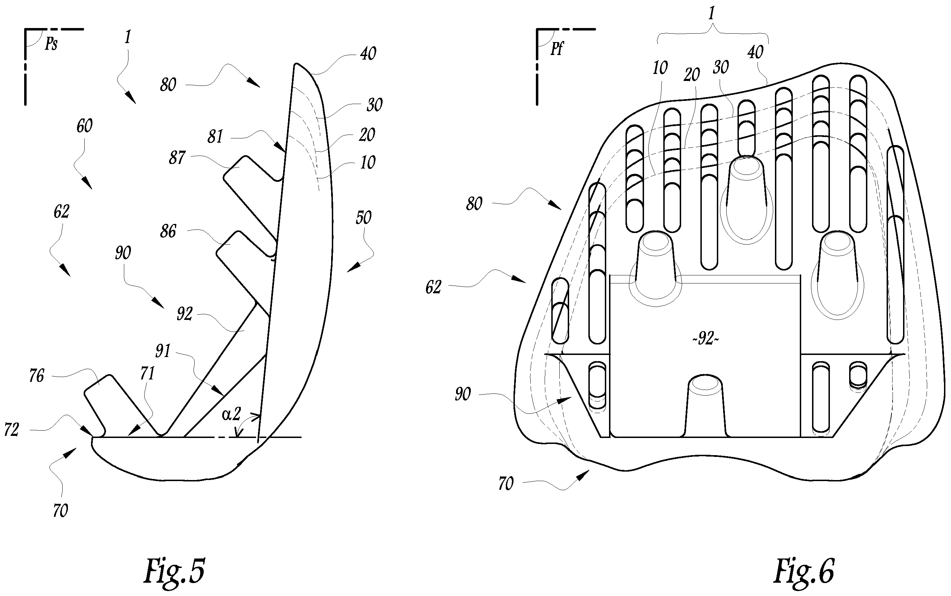

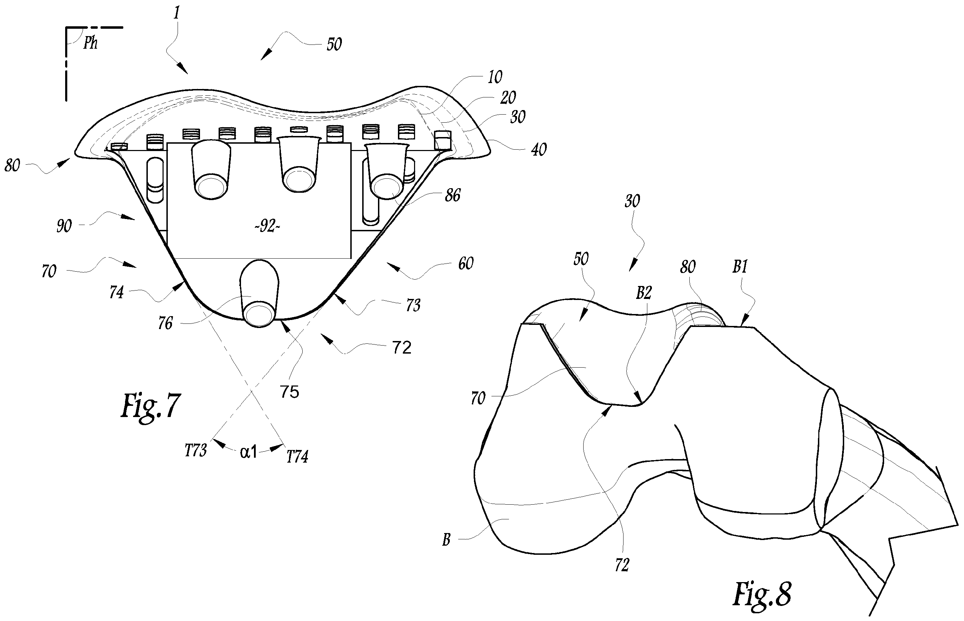

Cet implant fémoral 30 appartient à un ensemble 1 d'implants fémoraux 10, 20, 30 et 40, comme montré aux figures 5 à 7, destinés à équiper un os de fémur droit B d'un patient, comme montré à la figure 8.

En alternative, l'ensemble 1 peut comprendre des implants 10-40 adaptés pour équiper un os de fémur gauche d'un patient, ou des implants adaptés pour équiper sélectivement un fémur gauche et un fémur droit.

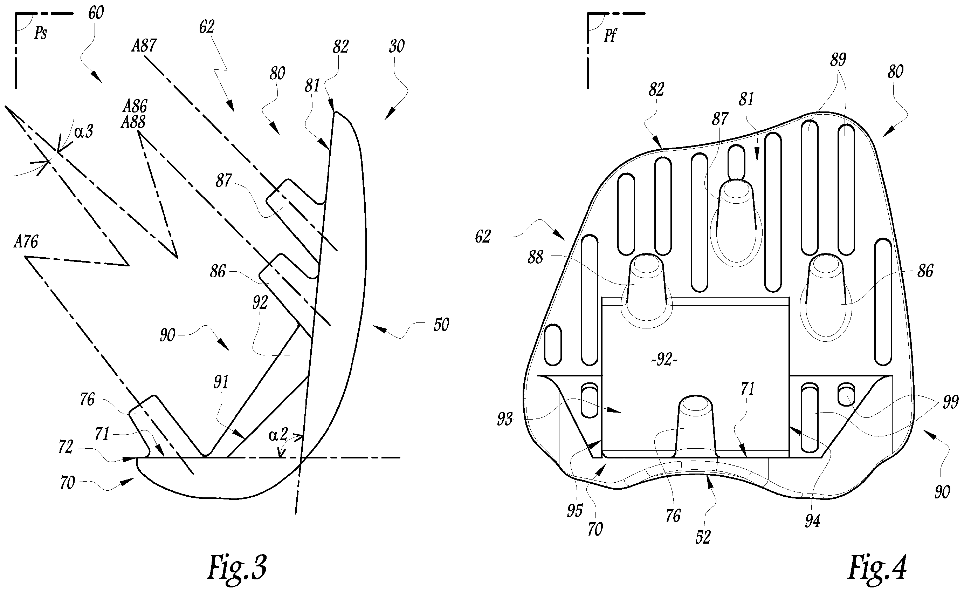

Dans la présente description, les conventions anatomiques sont en vigueur. En particulier, les figures 3 et 5 sont des vues dans un plan anatomique sagittal Ps, les figures 4 et 6 sont des vues dans un plan anatomique frontal Pf, tandis que la vue 7 est une vue dans un plan anatomique horizontal Ph.

Lors de la préparation de l'os du fémur B, le chirurgien réalise une préparation osseuse antérieure B1, en l'espèce une coupe osseuse, et une préparation osseuse distale B2, comme montré à la figure 8. Cette étape de préparation osseuse permet de définir des surfaces osseuses correspondant au profil et aux dimensions de l'implant fémoral à implanter. En outre, le chirurgien peut réaliser des coupes ou perçages additionnels de l'os B, non visibles à la figure 8, pour faciliter le positionnement et la fixation de l'implant sur l'os B.

En pratique, l'ensemble 1 comprend des implants 10, 20, 30 et 40 présentant des profils et dimensions différents, de sorte que le chirurgien peut sélectionner l'implant 10-40 le mieux adapté à l'anatomie du patient. Un tel ensemble 1 peut se présenter sous forme d'un kit chirurgical facilitant sa mise en oeuvre par le chirurgien. Par exemple, l'ensemble peut comprendre des moyens d'identification simple et pratique de chacun des implants 10-40 pour gagner du temps lors de l'opération chirurgicale. Cette identification peuvent être réalisées à l'aide d'un code couleur, de gravure, de marquage ou par tous autres moyens adaptés à la présente application.

Selon l'invention, l'ensemble 1 comprend au moins deux implants présentant des dimensions différentes. Sur l'exemple des figures 5 à 7, l'implant 10 présente les plus petites dimensions, l'implant 20 présente des dimensions supérieures à l'implant 10, l'implant 30 présente des dimensions supérieures à l'implant 20, et l'implant 40 présente les plus grandes dimensions.

La description ci-après est faite en référence à l'implant 30, étant entendu que les implants 10, 20 et 40 présentent une configuration comparable, quelles que soient leurs dimensions.

Comme montré aux figures 1 à 4, l'implant fémoral 30 comprend une face externe 50 adaptée pour coopérer avec un implant rotulien ou une rotule du patient, ainsi qu'une face interne 60 munie de moyens 62 d'ancrage à l'os B du patient, comme détaillé ci-après. La face externe 50 comporte une rainure de trochlée 52. L'implant 30 comprend également une partie distale 70 et une partie antérieure 80, chacune délimitée à la fois par la face externe 50 et la face interne 60. L'implant 30 comprend également une partie intermédiaire 90 reliant la partie distale 70 et la partie antérieure 80, du côté de la face interne 60.

La partie distale 70 de l'implant 30 comporte une surface interne distale 71 délimitée par une bordure 72, ainsi qu'un plot distal 76 formé en saillie de la surface 71. La bordure 72 est située globalement à la frontière distale postérieure entre la face externe 50 et la face interne 60 au niveau de la partie 70. Plus précisément, la bordure 72 est constituée par une bordure latérale 73 et une bordure médiale 74 reliées par une bordure postérieure 75. La bordure 72 de la partie distale 70 est destinée à affleurer la bordure de la préparation osseuse distale B2. Le plot 76 s'étend suivant un axe A76 depuis la surface 71 du côté de la face interne 60.

Dans le cadre de l'invention, les parties distales 70 des implants fémoraux 10-40 sont identiques, quelles que soient les dimensions des implants 10-40. En particulier, les surfaces 71, la bordure 72 et le plot 76 des implants 10-40 présentent les mêmes dimensions et le même agencement d'un implant 10-40 à l'autre. Ainsi, tous les implants 10-40 de l'ensemble 1 sont adaptés à la même préparation osseuse distale B2.

En outre, en projection dans un plan anatomique horizontal Ph, les bordures 73 et 74 présentent un profil sensiblement rectiligne. En d'autres termes, dans le plan Ph, les bordures 73 et 74 s'étendent suivant des droites, respectivement T73 et T74, tangentes à la partie distale 70. Dans le plan Ph, les tangentes T73 et T74 délimitent un angle distal α1 égal à 70 degrés sur l'exemple de la figure 7. En alternative, l'angle distal α1 peut être compris entre 60 degrés et 80 degrés, de préférence compris entre 68 degrés et 72 degrés. De préférence, comme montré à la figure 7, les angles distaux α1 des implants fémoraux 10-40 sont identiques, quelles que soient les dimensions de ces implants 10-40. Ainsi, l'implant 10-40 sélectionné par le chirurgien peut être positionné avec précision sur l'os B, avec une grande précision de l'affleurement entre la bordure de la préparation distale B2 et la bordure 72 de la partie distale 70.

La partie antérieure 80 de l'implant 30 comporte une surface interne frontale 81 délimitée par une bordure 82, ainsi que trois plots antérieurs 86, 87 et 88 formés en saillie de la surface 81. La bordure 82 est située globalement à la frontière supérieure entre la face externe 50 et la face interne 60 au niveau de la partie 80. La bordure 82 de la partie antérieure 80 est destinée à affleurer la bordure de la coupe osseuse antérieure B1. Les trois plots 86, 87 et 88 s'étendent respectivement suivant des axes A86, A87 et A88 depuis la surface 71 du côté de la face interne 60. Les axes A86, A87 et A88 sont parallèles entre eux. Des rainures 89 sont ménagées dans l'implant 30 au niveau de la surface 81.

Comme montré sur les figures 5 à 7, les parties antérieures 80 des différents implants 10-40 présentent des dimensions différentes. Autrement dit, les surfaces 81 et les bordures 82 présentent des dimensions différentes d'un implant 10-40 à l'autre. Ainsi, le chirurgien peut choisir l'implant 10-40 dont la partie antérieure 80 présente le profil le mieux adapté au profil de l'os B et notamment à la coupe antérieure B1. De même, les rainures 89 peuvent présenter une configuration différente pour chaque implant 10-40. En revanche, de préférence, comme sur l'exemple des figures 5 à 7, les plots 86, 87 et 88 présentent des dimensions et un positionnement relatif qui sont identiques, quelles que soient les dimensions des implants 10-40. Ainsi, la préparation de l'ancrage osseux est identique pour tous les implants 10-40 de l'ensemble 1.

Comme montré à la figure 3, la surface 71 et la surface 81 sont inclinées l'une par rapport à l'autre selon un angle obtus α2 compris entre 90 degrés et 100 degrés, de préférence entre 94 degrés et 96 degrés, en projection dans le plan anatomique sagittal Ps. De préférence, comme montré à la figure 5, cet angle α2 est identique pour tous les implants 10-40 de l'ensemble 1.

La partie intermédiaire 90 comporte une surface interne intermédiaire 91 reliant les surfaces 71 et 81, ainsi qu'un bloc chanfrein intercondylien 92 formé en saillie sur la face interne 60. Le bloc chanfrein 92 comporte une surface chanfrein 93, un bord latéral 94 et un bord médial 95. La surface chanfrein 93 du bloc 92 est plane et plus inclinée vers la partie distale 70 que la surface intermédiaire 91. Les bords 94 et 95 s'étendent à angle droit de la surface 91. Le bloc 92 s'étend en partie sur les surfaces 71 et 91. Des rainures 99 sont ménagées dans l'implant 30 au niveau de la surface 91.

De préférence, comme sur l'exemple des figures 5 à 7, les parties intermédiaires 90 des implants 10-40 présentent des dimensions et un positionnement par rapport à la partie distale 70 qui sont identiques, quelles que soient les dimensions de ces implants 10-40. Autrement dit, les parties intermédiaires 90 des implants 10-40 sont identiques, excepté les rainures 99 qui peuvent présenter une configuration différente pour chaque implant 10-40.

Sur l'exemple des figures, les moyens d'ancrage 62 de chaque implant 10-40 comprennent les plots 76, 86, 87 et 88, ainsi que le bloc chanfrein 92. Ces moyens 62 permettent de fixer rigidement l'implant 10-40 à l'os B du patient, lorsque les parties distale 70 et antérieure 80 sont positionnées respectivement contre les préparations distale B2 et antérieure B1. A cet effet, lors de la préparation osseuse, le chirurgien réalise des logements, perçages ou évidements dans l'os B, adaptés pour recevoir chacun des moyens d'ancrage 62 de l'implant 10-40.

Comme montré notamment aux figures 3 et 4, les plots 76, 86, 87 et 88 présentent chacun une forme cylindrique convergente en s'éloignant de la face interne 60, de manière à pouvoir pénétrer facilement les logements prévus à cet effet dans l'os B. Le plot distal 76 d'une part, et les plots antérieurs 86, 87 et 88 d'autre part, sont inclinés les uns vers les autres du côté de la face interne 60, selon un angle aigu α3 compris entre 5 degrés et 15 degrés en projection dans le plan anatomique sagittal Ps. Ainsi, lorsque l'implant 30 est ancré dans l'os B, la présence de cet angle α3 renforce la résistance de l'ancrage de la prothèse.

De préférence, comme sur l'exemple des figures 5 à 7, les moyens d'ancrage 62 des implants fémoraux 10-40 présentent des dimensions et un positionnement relatif identiques, quelles que soient les dimensions de ces implants 10-40. Ainsi, la préparation de l'ancrage osseux est identique pour tous les implants 10-40 de l'ensemble 1.

Lorsque le chirurgien a choisi l'implant 10-40 dont le profil et les dimensions sont les mieux adaptés à l'anatomie de l'os B, par exemple l'implant 30 sur la figure 8, l'intervention chirurgicale peut comprendre une étape de préparation de la face interne 60 de l'implant 30 à l'aide d'un ciment osseux chirurgical. Le ciment est réparti sur les surfaces 71, 81 et 91, sur les surfaces 93, 94 et 95 du bloc chanfrein 92, dans les rainures 89 et 99, ainsi que sur les plots 76, 86, 87 et 88. En alternative, seulement certaines parties de la face interne 60 reçoivent du ciment. Les rainures 89 et 99 sont prévues pour obtenir des volumes de ciment améliorant la résistance mécanique au cisaillement de l'implant 30 au niveau de la surface 81 ou 91 correspondante. En complément, du ciment peut également être déposé directement sur les surfaces de préparation osseuse B1 et B2.

Lors du positionnement et de la fixation de l'implant 30 sur l'os B, les moyens d'ancrage 62 pénètrent dans l'os B, notamment par impaction de l'implant 30. Ensuite, le ciment chirurgical interposé entre la face interne 60 et les surfaces de préparation osseuse B1 et B2 est comprimé entre l'implant 30 et l'os B. Le ciment pénètre dans l'os spongieux B, ce qui améliore l'ancrage de l'implant 30. L'intervention chirurgicale comporte alors une étape de séchage et prise du ciment. En complément, l'intervention peut également comprendre une étape de finition et/ou de contrôle et/ou d'équilibrage et/ou de test.

Par ailleurs, les implants 10-40 de l'ensemble 1 peuvent être conformés différemment des figures 1 à 8 sans sortir du cadre de l'invention.

En variante non représentée, les moyens d'ancrage 62 peuvent être conformés différemment des figures. De préférence, les moyens d'ancrage 62 comprennent au moins un plot distal 76 et au moins un plot antérieur 86, 87 et/ou 88 formés du côté de la face interne 60.

Selon une autre variante non représentée, la partie intermédiaire 90 peut comporter une nervure de rigidité en complément ou en alternative du bloc chanfrein 92.

Selon une autre variante non représentée, les surfaces 71, 81 et/ou 91 peuvent présenter des moyens différents de réception de ciment. Par exemple, les rainures 89 et 99 peuvent être réparties de manière différente. Selon un autre exemple, les surfaces 71, 81 et/ou 91 peuvent comporter des cuvettes de réception de ciment, qui peuvent être munies de reliefs tels que des pyramides, nervures ou rainures.

Selon une autre variante non représentée, chaque implant 10-40 appartenant à l'ensemble 1 peut être symétrique. Néanmoins, de préférence, chaque implant 10-40 appartenant à l'ensemble 1 est unicompartimental et asymétrique. Quel que soit le mode de réalisation, les parties distales 70 des implants fémoraux 10-40 sont identiques, quelles que soient les dimensions de ces implants 10-40. De préférence, les implants 10-40 comportent également d'autres parties dont les dimensions et le positionnement relatif sont identiques, à savoir les moyens d'ancrage 62, la partie intermédiaire 90 et notamment son bloc chanfrein 92.

L'ensemble d'implants fémoraux peut être adapté en termes de coût, de fonctionnalité et de performance.

## Revendications

1. Ensemble (1) d'implants fémoraux (10, 20, 30, 40) pour une prothèse de genou, comprenant au moins deux implants fémoraux (10, 20, 30, 40) présentant des dimensions différentes les unes des autres, chaque implant fémoral (10 ; 20 ; 30 ; 40) comprenant :
- une face externe (50) adaptée pour coopérer avec un implant rotulien ou une rotule,
- une face interne (60) munie de moyens (62) d'ancrage à un os (B) du patient, notamment avec interposition de ciment entre la face interne (60) et l'os (B),
- ainsi qu'une partie distale (70) et une partie antérieure (80) chacune délimitée à la fois sur la face externe (50) et la face interne (60), la partie distale (70) étant positionnable contre une préparation osseuse distale (B2), et la partie antérieure (80) étant positionnable contre une préparation osseuse antérieure (B1) ;
la partie distale (70) comportant une surface interne distale (71) délimitée par une première bordure (72) destinée à affleurer une bordure de la préparation osseuse distale (B2), la partie antérieure (80) comportant une surface interne frontale (81) délimitée par une seconde bordure (82) destinée à affleurer une bordure de la préparation osseuse antérieure (B1),
**caractérisé en ce qu'**au moins les parties distales (70) des implants fémoraux (10, 20, 30, 40) sont identiques, quelles que soient les dimensions des implants fémoraux (10, 20, 30, 40) et **en ce que** la partie distale (70) de chaque implant fémoral (10 ; 20 ; 30 ; 40) comporte une bordure latérale (73) et une bordure médiale (74) qui, en projection dans un plan anatomique horizontal (Ph), s'étendent suivant des droites (T73, T74) délimitant un angle distal (α1) compris entre 60 degrés et 80 degrés, de préférence compris entre 68 degrés et 72 degrés, en particulier égal à 70 degrés, les angles distaux (α1) des implants fémoraux (10, 20, 30, 40) étant identiques, quelles que soient les dimensions des implants fémoraux (10, 20, 30, 40).

2. Ensemble (1) d'implants fémoraux (10, 20, 30, 40) selon la revendication 1, **caractérisé en ce que** les moyens d'ancrage (62) des implants fémoraux (10, 20, 30, 40) présentent des dimensions et un positionnement relatif qui sont identiques, quelles que soient les dimensions des implants fémoraux (10, 20, 30, 40).

3. Ensemble (1) d'implants fémoraux (10, 20, 30, 40) selon l'une des revendications précédentes, **caractérisé en ce que** chaque implant fémoral (10 ; 20 ; 30 ; 40) comprend une partie intermédiaire (90) reliant la partie distale (70) et la partie antérieure (80) du côté de la face interne (60), les parties intermédiaires (90) des implants fémoraux (10, 20, 30, 40) présentant des dimensions et un positionnement par rapport à la partie distale (70) qui sont identiques, quelles que soient les dimensions des implants fémoraux (10, 20, 30, 40).

4. Ensemble (1) d'implants fémoraux (10, 20, 30, 40) selon la revendication 3, **caractérisé en ce que** pour chaque implant fémoral (10 ; 20 ; 30 ; 40), la partie intermédiaire (90) comporte une surface intermédiaire (91) et un bloc chanfrein (92) qui est plus incliné vers la partie distale (70) que la surface intermédiaire (91).

5. Ensemble (1) d'implants fémoraux (10, 20, 30, 40) selon l'une des revendications précédentes, **caractérisé en ce que** pour chaque implant fémoral (10 ; 20 ; 30 ; 40), les moyens d'ancrage (62) comprennent au moins un plot distal (76) et au moins un plot antérieur (86, 87, 88) qui sont formés du côté de la face interne (60), respectivement sur la partie distale (70) et sur la partie antérieure (80), les plots (76, 86, 87, 88) des implants fémoraux (10, 20, 30, 40) présentant des dimensions et un positionnement relatif qui sont identiques, quelles que soient les dimensions des implants fémoraux (10, 20, 30, 40).

6. Ensemble (1) d'implants fémoraux (10, 20, 30, 40) selon la revendication 5, **caractérisé en ce que** pour chaque implant fémoral (10 ; 20 ; 30 ; 40), le plot distal (76) et le plot antérieur (86 ; 87 ; 88) sont inclinés l'un vers l'autre du côté de la face interne (60) selon un angle aigu (α3) compris entre 5 degrés et 15 degrés en projection dans un plan anatomique sagittal (Ps).

7. Ensemble (1) d'implants fémoraux (10, 20, 30, 40) selon l'une des revendications précédentes, **caractérisé en ce que** pour chaque implant fémoral (10 ; 20 ; 30 ; 40), les moyens d'ancrage (62) comportent trois plots antérieurs (86, 87, 88) ayant des axes (A86, A87, A88) parallèles entre eux.

8. Ensemble (1) d'implants fémoraux (10, 20, 30, 40) selon l'une des revendications précédentes, **caractérisé en ce que** chaque implant fémoral (10 ; 20 ; 30 ; 40) comporte une surface interne distale (71) et une surface interne frontale (81) qui sont inclinées l'une par rapport à l'autre selon un angle obtus (α2) compris entre 90 degrés et 100 degrés, de préférence entre 94 degrés et 96 degrés, en projection dans un plan anatomique sagittal (Ps).

9. Ensemble (1) d'implants fémoraux (10, 20, 30, 40) selon l'une des revendications précédentes, **caractérisé en ce que** chaque implant fémoral (10 ; 20 ; 30 ; 40) est unicompartimental et asymétrique.

## Patentansprüche

1. Anordnung (1) von Oberschenkelimplantaten (10, 20, 30, 40) für eine Knieprothese, umfassend mindestens zwei Oberschenkelimplantate (10, 20, 30, 40) mit unterschiedlichen Abmessungen voneinander, wobei jedes Oberschenkelimplantat (10; 20; 30; 40) umfasst:
- eine Außenfläche (50), die geeignet ist, um mit einem Kniescheibenimplantat oder einer Kniescheibe zusammenzuwirken,
- eine Innenfläche (60), die mit Mitteln (62) zum Verankern an einem Knochen (B) des Patienten versehen ist, insbesondere mit Hilfe von Zement, der zwischen der Innenfläche (60) und dem Knochen (B) eingebracht ist,
- sowie einen distalen Abschnitt (70) und einen vorderen Abschnitt (80), die jeweils sowohl auf der äußeren (50) als auch auf der inneren (60) Fläche begrenzt sind, wobei der distale Abschnitt (70) gegen eine distale Knochenpräparation (B2) positionierbar ist und der vordere Abschnitt (80) gegen eine vordere Knochenpräparation (B1) positionierbar ist;
wobei der distale Teil (70) eine distale Innenfläche (71) umfasst, die durch einen ersten Rand (72) zum Bündigmachen mit einem Rand der distalen Knochenpräparation (B2) begrenzt ist, wobei der vordere Abschnitt (80) eine frontale Innenfläche (81) aufweist, die durch einen zweiten Rand (82) zum Bündigmachen mit einem Rand der vorderen Knochenpräparation (B1) begrenzt ist, **dadurch gekennzeichnet, dass** mindestens die distalen Abschnitte (70) der Oberschenkelimplantate (10, 20, 30, 40) unabhängig von den Abmessungen der Oberschenkelimplantate (10, 20, 30, 40) identisch sind **und dass** der distale Abschnitt (70) jedes Oberschenkelimplantats (10; 20; 30; 40) einen seitlichen Rand (73) und einen medialen Rand (74) aufweist, die sich, wenn sie in einer horizontalen anatomischen Ebene (Ph) projiziert werden, entlang gerader Linien (T73, T74) erstrecken, die einen distalen Winkel (α1) zwischen 60 Grad und 80 Grad, vorzugsweise zwischen 68 Grad und 72 Grad, insbesondere von exakt 70 Grad, begrenzen, wobei die distalen Winkel (α1) der Oberschenkelimplantate (10, 20, 30, 40) unabhängig von den Abmessungen der Oberschenkelimplantate (10, 20, 30, 40) identisch sind.

2. Anordnung (1) von Oberschenkelimplantaten (10, 20, 30, 40) nach Anspruch 1, **dadurch gekennzeichnet, dass** die Verankerungsmittel (62) der Oberschenkelimplantate (10, 20, 30, 40) Abmessungen und eine relative Positionierung aufweisen, die unabhängig von den Abmessungen der Oberschenkelimplantate (10, 20, 30, 40) identisch sind.

3. Anordnung (1) von Oberschenkelimplantaten (10, 20, 30, 40) nach einem der vorstehenden Ansprüche, **dadurch gekennzeichnet, dass** jedes Oberschenkelimplantat (10; 20; 30; 40) einen Zwischenabschnitt (90) umfasst, der den distalen Abschnitt (70) und den vorderen Abschnitt (80) auf der Innenseite (60) verbindet, wobei die Zwischenabschnitte (90) der Oberschenkelimplantate (10, 20, 30, 40) Abmessungen und eine Positionierung in Bezug auf den distalen Abschnitt (70) aufweisen, die unabhängig von den Abmessungen der Oberschenkelimplantate (10, 20, 30, 40) identisch sind.

4. Anordnung (1) von Oberschenkelimplantaten (10, 20, 30, 40) nach Anspruch 3, **dadurch gekennzeichnet, dass** der Zwischenabschnitt (90) für jedes Oberschenkelimplantat (10; 20; 30; 40) eine Zwischenfläche (91) und einen Fasenblock (92) aufweist, der stärker zum distalen Abschnitt (70) geneigt ist als die Zwischenfläche (91).

5. Anordnung (1) von Oberschenkelimplantaten (10, 20, 30, 40) nach einem der vorstehenden Ansprüche, **dadurch gekennzeichnet, dass** für jedes Oberschenkelimplantat (10; 20; 30; 40), wobei die Verankerungsmittel (62) mindestens einen distalen Bolzen (76) und mindestens einen vorderen Bolzen (86, 87, 88) umfassen, die auf der Innenseite (60) jeweils auf dem distalen Abschnitt (70) und auf dem vorderen (80) Abschnitt ausgebildet sind, wobei die Bolzen (76, 86, 87, 88) der Oberschenkelimplantate (10, 20, 30, 40) Abmessungen und eine relative Positionierung aufweisen, die unabhängig von den Abmessungen der Oberschenkelimplantate (10, 20, 30, 40) identisch sind.

6. Anordnung (1) von Oberschenkelimplantaten (10, 20, 30, 40) nach Anspruch 5, **dadurch gekennzeichnet, dass** für jedes Oberschenkelimplantat (10; 20; 30; 40) der distale Bolzen (76) und der vordere Bolzen (86; 87; 88) auf der Innenseite (60) in einem spitzen Winkel (α3) zwischen 5 Grad und 15 Grad in einer anatomischen Sagittalebene (Ps) in Projektion zueinander geneigt sind.

7. Anordnung (1) von Oberschenkelimplantaten (10, 20, 30, 40) nach einem der vorstehenden Ansprüche, **dadurch gekennzeichnet, dass** die Verankerungsmittel (62) für jedes Oberschenkelimplantat (10; 20; 30; 40) drei vordere Bolzen (86, 87, 88) mit parallel zueinander verlaufenden Achsen (A86, A87, A88) umfassen.

8. Anordnung (1) von Oberschenkelimplantaten (10, 20, 30, 40) nach einem der vorstehenden Ansprüche, **dadurch gekennzeichnet, dass** jedes Oberschenkelimplantat (10; 20; 30; 40) eine distale Innenfläche (71) und eine frontale Innenfläche (81) aufweist, die in einem stumpfen Winkel (α2) zwischen 90 Grad und 100 Grad, vorzugsweise zwischen 94 Grad und 96 Grad, in einer anatomischen Sagittalebene (Ps) in Projektion zueinander geneigt sind.

9. Anordnung (1) von Oberschenkelimplantaten (10, 20, 30, 40) nach einem der vorstehenden Ansprüche, **dadurch gekennzeichnet, dass** jedes Oberschenkelimplantat (10; 20; 30; 40) unikondylär und asymmetrisch ist.

## Claims

1. Set (1) of femoral implants (10, 20, 30, 40) for a knee prosthesis, comprising at least two femoral implants (10, 20, 30, 40) having different dimensions from one another, each femoral implant (10; 20; 30; 40) comprising:
- an outer face (50) suitable for cooperating with a patellar implant or a patella,
- an inner face (60) provided with means (62) for anchoring to a bone (B) of the patient, in particular so as to have cement inserted between the inner face (60) and the bone (B),
- and a distal portion (70) and an anterior portion (80) each delimited on both the outer face (50) and the inner face (60), it being possible to position the distal portion (70) against a distal bone preparation (B2), and it being possible to position the anterior portion (80) against an anterior bone preparation (B1);
the distal portion (70) comprising a distal inner surface (71) delimited by a first edge (72) intended to be flush with an edge of the distal bone preparation (B2), the anterior portion (80) comprising a front inner surface (81) delimited by a second edge (82) intended to be flush with an edge of the anterior bone preparation (B1), **characterized in that** at least the distal portions (70) of the femoral implants (10, 20, 30, 40) are identical irrespective of the dimensions of the femoral implants (10, 20, 30, 40), and **in that** the distal portion (70) of each femoral implant (10; 20; 30; 40) has a lateral edge (73) and a medial edge (74) which, projected in a horizontal anatomical plane (Ph), extend along lines (T73, T74) delimiting a distal angle (α1) of between 60 degrees and 80 degrees, preferably between 68 degrees and 72 degrees, in particular equal to 70 degrees, the distal angles (α1) of the femoral implants (10, 20, 30, 40) being identical irrespective of the dimensions of the femoral implants (10, 20, 30, 40).

2. Set (1) of femoral implants (10, 20, 30, 40) according to claim 1, **characterized in that** the anchoring means (62) of the femoral implants (10, 20, 30, 40) have dimensions and relative positioning which are identical irrespective of the dimensions of the femoral implants (10, 20, 30, 40).

3. Set (1) of femoral implants (10, 20, 30, 40) according to either of the preceding claims, **characterized in that** each femoral implant (10; 20; 30; 40) comprises an intermediate portion (90) connecting the distal portion (70) and the anterior portion (80) on the inner face (60), the intermediate portions (90) of the femoral implants (10, 20, 30, 40) having dimensions and positioning relative to the distal portion (70) which are identical irrespective of the dimensions of the femoral implants (10, 20, 30, 40).

4. Set (1) of femoral implants (10, 20, 30, 40) according to claim 3, **characterized in that**, for each femoral implant (10; 20; 30; 40), the intermediate portion (90) has an intermediate surface (91) and a chamfer block (92) which is more inclined toward the distal portion (70) than the intermediate surface (91).

5. Set (1) of femoral implants (10, 20, 30, 40) according to any of the preceding claims, **characterized in that**, for each femoral implant (10; 20; 30; 40), the anchoring means (62) comprise at least one distal pad (76) and at least one anterior pad (86, 87, 88) which are formed on the inner face (60), respectively on the distal portion (70) and on the anterior portion (80), the pads (76, 86, 87, 88) of the femoral implants (10, 20, 30, 40) having dimensions and relative positioning which are identical irrespective of the dimensions of the femoral implants (10, 20, 30, 40).

6. Set (1) of femoral implants (10, 20, 30, 40) according to claim 5, **characterized in that**, for each femoral implant (10; 20; 30; 40), the distal pad (76) and the anterior pad (86; 87; 88) are inclined toward one another on the inner face (60) at an acute angle (α3) of between 5 degrees and 15 degrees, projected in a sagittal anatomical plane (Ps).

7. Set (1) of femoral implants (10, 20, 30, 40) according to any of the preceding claims, **characterized in that**, for each femoral implant (10; 20; 30; 40), the anchoring means (62) comprise three anterior pads (86, 87, 88) having axes (A86, A87, A88) parallel to one another.

8. Set (1) of femoral implants (10, 20, 30, 40) according to any of the preceding claims, **characterized in that** each femoral implant (10; 20; 30; 40) has a distal inner surface (71) and a front inner surface (81) which are inclined relative to one another at an obtuse angle (α2) of between 90 degrees and 100 degrees, preferably between 94 degrees and 96 degrees, projected in a sagittal anatomical plane (Ps).

9. Set (1) of femoral implants (10, 20, 30, 40) according to any of the preceding claims, **characterized in that** each femoral implant (10; 20; 30; 40) is unicompartmental and asymmetrical.
